# EUROPEAN PATENT SPECIFICATION

(11) **EP 4 539 908 B1**
(45) Date of publication and mention of the grant of the patent: **09.09.2026**
(21) Application number: 23732572.5
(22) Date of filing: 14.06.2023
(51) Int. Cl.: A61M 5/24, A61M 5/315

(54) **AN APPARATUS FOR SUBCUTANEOUS DELIVERY OF A MEDICAMENT**
VORRICHTUNG ZUR SUBKUTANEN ABGABE EINES MEDIKAMENTS
APPAREIL D'ADMINISTRATION SOUS-CUTANÉE D'UN MÉDICAMENT

(30) Priority: 14.06.2022 US 202263351857 P; 01.09.2022 EP 22193553
(43) Date of publication of application: 23.04.2025
(73) Proprietor: SHL Medical AG, 6302 Zug (CH)
(72) Inventor: RAVAYNIA, Paolo, 6302 Zug (CH); EGLOFF, Christoph, 6302 Zug (CH); WANG, Hsuan, 6302 Zug (CH)
(86) International application number: PCT/EP2023/065947
(87) International publication number: WO 2023/242261

(56) References cited:
- US-A1- 2005 065 466
- US-A1- 2020 254 182

## Description

### Technical field

The present disclosure relates to the technical field of administration of therapeutical medicaments. In particular, the present disclosure relates to the technical field of subcutaneous administration of single large-volume medications.

### Background

The service of relocating therapeutic care from clinic to the home of the patient is becoming increasingly popular. This enables the patient to receive the necessary treatment in their home's instead of having to spend lengthy visits at the hospital.

This could be beneficial since the patient may feel more comfortable and safe receiving care in a home environment, being closer to their family and relatives, and the occupancy of the hospitals can be reduced. Some devices can be found in US2020/254182 and US2005/065466, for example.

However, many new biologic medications are being formulated for much larger volumes out of necessity or for competitive advantage. For these drugs, intravenous (IV) administration is currently the standard of care. In general, the treatment of using these drugs contains multiple medications that need to be administrated in sequence. Therefore, the IV injection needs to be administrated by a professional health carer/nurse and it is hard to be administrated at home by patients themselves.

Furthermore, pharma companies are investing to transition a range of medications from IV to subcutaneous (SC) delivery. However, the existed large volume SC injectors need a lot of complex steps for setting and changing the delivery of different medications for the patients. The current SC delivery options are thus not adapted for use in a home environment.

Thus, there is a need for improved SC delivery devices for high-volume medications.

### Summary

It is realized as a part of the present disclosure that a number of complex steps for delivering medications to a patient reduces the possibility of treating the patient at home. This could cause further stress with the treated patient, having to go back and forth to a hospital, and not being able to properly relax and recover.

It is further realized as a part of the present disclosure that having to rely on a professional health carer for administration of medications is another reason preventing patients from being treated at home. This is partly due to the fact that the health carer can ensure that the correct dose is delivered, and that the environment is kept sterile. It could become very costly to employ professional health carers to make home visits on a regular basis, why it is not a widely used or even accessible option for most patients.

Therefore, the inventors have come to the realization that there is a need for an apparatus that permits a patient to self-administrate a fixed dose of a medication in a sterile manner at home.

The present disclosure therefore provides an apparatus for delivery of a subcutaneous medicament and a method for subcutaneous delivery of a medicament in the independent claims, that at least partially mitigates the above-mentioned drawbacks. Preferred embodiments are defined in the dependent claims.

Hence, according to a first aspect of the present disclosure, there is provided an apparatus for delivery of a medicament. The apparatus comprises a flexible container containing the medicament; a delivery unit comprising; a first plate extending along a longitudinal axis. The first plate comprises at least one first magnet; a second plate extending along a longitudinal axis. The first plate; wherein the second plate comprises at least one second magnet. A portion of the flexible container is positioned between the first plate and the second plate in a direction transverse to the longitudinal axis. A delivery actuating device movably arranged between a first position where the delivery actuating device holds the first and second plate, such that the flexible container is prevented from being pressed between the first plate and the second plate and a delivery position where the delivery actuating device releases the first and second plate in the first position, such that the flexible container can be pressed between the first plate and the second plate.

In a second aspect of the present disclosure, a method for delivering a medicament using the apparatus according to the first aspect of the disclosure is provided. The method comprises arranging a flexible container containing a medicament in the apparatus.

The method further comprises installing the apparatus in the first position, i.e. the position wherein the delivery actuating unit is at least partially engaged with the first and second plates. The plates are thus kept in a separated state, preventing the plates to move towards each other.

Further, the method comprises triggering the delivery actuating unit. The triggering of the delivery actuating unit may for example be done by the patient themselves, a family member or a nurse. The delivery actuating unit is movably arranged between the first position and a delivery position. Triggering of the delivery actuating unit thus means moving the delivery actuating unit from the first position to the delivery position. In the delivery position, the delivery actuating unit releases the first and second plates, such that they are free to move towards each other, and apply pressure on the flexible container sandwiched therebetween.

The method further comprises activating a delivery of the medicament. The medicament may be delivered to a patient. The medicament may be delivered subcutaneously. The medicament may also be delivered in other ways suitable for the type of treatment the patient receives. Further, the medicament may be delivered in a manner corresponding to that of a conventional auto-injector. The apparatus may be connected to any of the above means of delivery, such that the medicament being pressed out from the flexible container may be delivered directly to the patient without intermediate steps.

By the term "flexible container" is herein meant a container suitable for storing a medicament, and which shape or contours are affected by the enclosed medicament in the filled, sealed state. A flexible container could for example be a plastic bag, a silicone bag, a bioplastic bag, or any other flexible material suitable for holding a medicament.

Thus, there is provided an apparatus and a method for subcutaneous delivery of a medicament. The apparatus allows for self-administration of a medicament at home. The apparatus consists of parts that are purely mechanical, for example the first and second plates and the flexible container. This is advantageous since the apparatus does not require any electronic equipment in order to work, which may increase the usability and accessibility for patients not having unlimited access to electricity. A further advantage may be that the apparatus may be designed to be portable and wireless. Furthermore, the absence of electronics may ensure that the apparatus can deliver the medicament in a more silent manner, not spreading any noise or other unwelcoming sounds.

In one example, the first and second plate have a substantially rectangular (observe in the direction transverse to the longitudinal axis) shape, with a top surface and a bottom surface. The plates may be made of a non-magnetic material, such as a type of plastic, bioplastic, or similar.

According to an embodiment of the present disclosure, the first and/or second plate is made of a transparent material. The flexible container may be sandwiched between a bottom surface of the first plate and a top surface of the second plate, or vice versa. Having the plates made of a transparent material may be advantageous in that it increases the drug visibility. Since the flexible container may be sandwiched between the first and second plates in the direction transverse to the longitudinal axis, the flexible container may be visible through the plates. Thus, a professional health carer or the patient, may clearly see what type of medicament that is arranged in the apparatus. This may be important during arrangement of the apparatus, delivery of the apparatus to a patient, or during inspection of the apparatus by an authority or the like.

In one example, the flexible container has a substantially rectangular cross-sectional shape, viewed from a top or a bottom. Thus, the shape of the top and/or bottom surface of the first and second plates may conform with a shape of a top and/or bottom cross-sectional shape of the flexible container. A contact surface between the plates and the flexible container may therefore be increased. An increased contact surface may increase the pressure from the plates exerted on the flexible container, thereby the medicament may be pressed out from the flexible container in a more effective way. If the contact surface is decreased between the plates and the flexible container, medicament may get stuck inside the flexible container, not being delivered to the patient.

In another example, the size of the first and second plates is greater than the size of the flexible container, such that the plates cover the flexible container in its entirety. This may ensure that all the medicament inside the flexible container is pressed out and delivered.

Further, the first and second plates comprises at least one magnet. According to an embodiment of the present disclosure, the at least one first magnet and the at least one second magnet have opposite magnetic poles and configured to attract one another. Thus, there is a magnetic force present between the magnets, causing the magnets to attract each other, and therefore the first and second plates to move towards each other. The plates moving towards each other in its turn exerts a pressure on the flexible container, causing the medicament to be pressed out of the flexible container and delivered to a patient. When the distance between the plates decreases, the magnetic force between the magnets may increase, since the opposite poles moves closer to each other. Since the medicament inside the flexible container may be emptied over time as a result of the pressure from the plates, the counteracting pressure from the filled flexible container decreases. Therefore, an advantageous aspect of the present embodiment may be that the magnets in the plates applies a continuous force on the bag over time, why a continuous flow of the medicament may be delivered to the patient.

This embodiment is advantageous in that the delivery of the medicament may be carried out mechanically, without the need for any electronic devices. This may decrease the amount of steps needed in order to set the apparatus up, which increases the usability of the apparatus.

In one example, the magnets may have a size substantially corresponding to the size of the plates. In another example, the size of the magnets may be smaller than the size of the plates, such that a plurality of magnets may be arranged in the first and second plates, respectively.

According to an embodiment of the present disclosure, the at least one first magnet and the at least one second magnet are four magnets. Thus, there are four magnets arranged in each plate. The placement of the magnets on the plates may thus be chosen such that the magnetic force is distributed over a desired portion of the surface of the plates. This may be advantageous in that the apparatus is versatile and can be adapted to be suitable for a number of shapes and placements of the flexible containers. It may also be adapted in order to be suitable for different volumes of medicaments to be delivered. The strength of the magnetic force moving the plates towards each other may be increased with the increasing number of magnets arranged in the plates and/or the size of the magnets.

In one example, the magnets are arranged in corresponding positions in the first and second plates respectively. Thus, the magnetic force between the corresponding magnets may be increased, and the pressure exerted on the flexible container may be more evenly distributed.

According to an embodiment of the present disclosure, the four first and second magnets are arranged adjacent the corners of said first and second plates, in the case where the first and second plates has a substantially rectangular shape, or any other shape including corners. This may be advantageous in that the magnetic force may be evenly distributed over the whole surface of the plates, without covering the entire plate with a single magnet. Therefore, the weight-efficiency of the plates may be increased. A further advantage from present embodiment is that the drug visibility may be increased. The placement of the magnets in the corners of each plate permits the flexible container to be visible through the plates, in the case where the plates are made of a transparent material.

According to an embodiment of the present disclosure, the first and second magnets are permanent magnets. This may be advantageous in that the apparatus is mechanical, no electricity need to be provided, and the magnets does not require any configuration in order to provide a magnetic force.

In another embodiment, the at least one first and second magnets are electromagnetic coils.

Further, the delivery actuating device is configured to arranged in a first position where it is at least partially engaged with the first and second plates in order to keep the plates being spaced apart from the flexible container. In the example where the plates are substantially rectangular, the delivery actuating device may be arranged in one of the short ends of the plates. It is to be understood that there are other possible embodiments wherein the delivery actuating device may be arranged in different positions.

According to an embodiment of the present disclosure, the delivery actuating device further comprises a spacer at least partially arranged between the first and second plates in the direction transverse to the longitudinal axis when the delivery actuating device is in the first position; and wherein the spacer is spaced apart from the first plate and the second plate when the delivery actuating device is in the delivery position. In an exemplifying embodiment, the spacer is made of a non-magnetic material. The spacer for example be made of a hard plastic or bioplastic material, however other non-magnetic materials are possible.

In this embodiment, the delivery actuating device is configured to hold the first plate and the second plate with the spacer in the first position; and release the first plate and the second plate by moving the spacer.

According to an exemplifying embodiment, wherein the at least one first and second magnets are electromagnetic coils, the spacer may be made of a material with conductive capacities. Thus, according to an exemplifying embodiment, the delivery actuating device is electrically connected to the first and second plates. This is advantageous since the spacer may provide electrical current to control the magnetic field of the electromagnetic coils. The spacer may thus be arranged between the first and second plates in the first position to separate them.

According to an embodiment of the present disclosure, the first and second plates are arranged on opposite sides along a longitudinal axis. The delivery actuating device is arranged adjacent the delivery unit along the longitudinal axis. Further, the flexible container is arranged in-between the first and second plates along the longitudinal axis. The spacer thus separate the plates such that the distance to the longitudinal axis is substantially perpendicular. In one exemplifying embodiment, the plates are separated in one end by the spacer in the first position. This means that the short ends opposite to the ends where the spacer is may be arranged with no distance between them, i.e. in contact.

Thus, an advantageous aspect of the present embodiments may be that the apparatus can be installed in the first position before delivery to the patient, thus preventing the plates to move towards each other. The apparatus may then be shipped from the manufacturer and/or hospital maintaining said first position, thus decreasing the risk for the medicament to be pressed out when not connected to a receiving patient. A further advantage is that the apparatus can be configured beforehand, such that the only remaining step for the patient to carry out is to move the delivery actuating device from the first position to the delivery position. Thus, increasing the usability and safety of the apparatus.

According to an embodiment of the present disclosure, the delivery actuating device further comprises a releasing means configured to actuate a movement of the spacer relative the first and second plates, such that said plates are released from the first position. The releasing means may be directly or indirectly connected to the spacer, so as to trigger the movement from the first position to the delivery position.

In an exemplifying embodiment, the releasing means is a button connected to the spacer; and wherein the delivery actuating device is configured to be moved from the first position to the delivery position when the button is manually moved. In one example, the button is a push button. The push button may be pushed by a patient, a family member or a professional health carer, for example. The present embodiment is advantageous in that it may increase the usability of the apparatus, since there is only one step required in order to start delivering the medicament.

According to another embodiment of the present disclosure, the releasing means further comprises an elastic element extending in the direction of the longitudinal axis, between the delivery unit and the delivery actuating device; wherein the elastic element is configured to support the spacer in the first position. In an exemplifying embodiment, the elastic element is a spring, i.e. a compression spring. The elastic element may be arranged between the delivery actuating unit and the delivery unit. The elastic element may be in an unloaded state in the first position, and a compressed state in the delivery position. In one example, the elastic element extends in the direction of the longitudinal axis between a first end and a second end. The first end is adjacent to either the first plate or the second plate; and wherein the second end is adjacent to the spacer. A trigger of the releasing means may thus overcome a compression load in order to compress the elastic element, such that the spacer may be removed from between the first and second plates. This is advantageous in that the risk of accidentally releasing the plates decreases. For example, if the apparatus gets exposed to any outer impacts during transport, such as bumps or shaking, etc., the elastic element may prevent the apparatus from moving to the delivery position.

According to an embodiment of the present disclosure, the flexible container further comprises a sealed exit where the medicament can be expelled from the exit. In an exemplifying embodiment, the sealed exit comprises a one-way valve.

The sealed exit may be arranged adjacent to the spacer and the releasing means. This embodiment is advantageous in that the sealed exit may ensure that the medicament exits the flexible container in a predetermined place. Further, the sealed exit thus may prevent the flexible container from bursting (due to the pressure exerted from the plates) in an arbitrary placement on the flexible container, which may lead to the medicament leaking out between the plates instead of being delivered to a patient. The one-way valve may prevent the exited medicament from travelling back into the flexible container, ensuring that the medicament is delivered to the patient. The sealed exit thus may increase the overall reliability of the apparatus.

Additionally, in another example, the sealed exit comprises a pierceable seal.

According to an embodiment of the present disclosure, the delivery actuating device comprises a piercing element extending in the direction of the longitudinal axis. The piercing element is spaced apart from the flexible container when the delivery actuating device is in the first position; and wherein the piercing element is positioned into the flexible container when the delivery actuating device is in the delivery position.

The piercing element is configured to pierce the flexible container in the delivery position. In the example where the flexible container comprises the pierceable seal, the piercing element is configured to pierce the pierceable seal. In an exemplifying embodiment, the piercing element is a needle. The piercing element may be arranged in connection to the releasing means. For example, the piercing element may be mounted on the releasing means, such that upon actuation of the releasing means, the piercing element may move together with the releasing means to release the first and second plates, and simultaneously piercing the flexible container. In one example, the piercing element is attached to the button. Thus, the when the user manually moves the button to move the delivery actuating device from the first position to the delivery position, the spacer is moved out from its position between the first and second plates, and also push the needle into the sealed exit in the flexible container.

The present embodiment is advantageous in that the piercing element may provide a controlled way of letting the medicament out from the flexible container. Further, the size of the piercing element may advantageously be chosen in order to control a flow rate of the exiting medicament. A larger size of the piercing element may generate an increased flow rate of the exiting medicament. For example, a professional health carer or manufacturer of the medicament, may therefore adapt a size of the piercing element depending on the medicament, dose, or patients size (for example, a child may require a smaller flow rate of the same medicament than an adult).

Further, the piercing element may be sterile, and thus advantageously arranged in the apparatus to ensure that the medicament is being delivered in a sterile manner.

According to an embodiment of the present disclosure, the delivery actuating device further comprises a tube defining a medicament path from the flexible container to a patient, for delivery of the medicament through said tube. According to an exemplifying embodiment, the piercing element is arranged in the tube, such that the medicament is delivered through the tube upon release. The tube may define a medicament path between the flexible container and the patient intended to receive the medicament. This is advantageous in that the medicament may be led out from the flexible container through the tube and to the patient, preventing the medicament from coming in contact with the surrounding environment. Thus, the medicament path may be kept sterile. A further advantage may be that the tube may be transparent, such that the medicament flowing through the tube may be visible during delivery, ensuring the patient that the apparatus is operating as expected. The patient may also clearly see when the flexible container is emptied, i.e. the delivery of the medicament is completed, which increases the reliability of the apparatus.

According to an embodiment of the present disclosure, the apparatus further comprises a sensor arranged to monitor and determine a flow rate of the medicament flowing out from the dosing apparatus. The sensor may for example be a flowmeter, a calorimetric sensor, pinwheel sensor, ultrasound sensor or similar.
According to a further embodiment of the present disclosure, the apparatus further comprises an electromechanical valve configured to, based on information from the sensor regarding the flow rate of the medicament, open or close depending on a preset threshold value of the flow rate. According to an exemplifying embodiment, the sensor and the electromechanical valve are arranged on the tube. The electromechanical valve may for example be a pinch valve or a solenoid valve. The present embodiments are advantageous in that further controllability of the apparatus may be provided. For example, upon installation of the apparatus, a professional health carer or manufacturer may preset a flow rate value based on the type of medicament to be delivered. Upon delivery to the patient, the sensor and the electromechanical valve may control a flow rate to correspond to the preset value determined during installation. Thus, the reliability of the apparatus may be ensured, such that the patient receives the medicament in the desired way. For example, a preferred flow rate may be 1ml/min.

In one exemplifying embodiment, the apparatus further comprises a frame, configured to at least partially enclose the first and second plates, the flexible container and the delivery actuation unit. The frame may protect the enclosed components during transportation, and maintain the position in which they were installed. The frame may also provide a way of ensuring that the patient cannot reach the medication other than by delivering it the intended way. This may also be a safety measure for the patient, knowing that no middleman could have manipulated the medication or exchanged the flexible container during transportation.

According to an exemplifying embodiment, the frame may also provide a sterile environment for the enclosed components. A sterile environment is important considering that the medicament to be delivered is a medicament, and that the way of delivery may be subcutaneous, why delivering a contaminated medication may put the patient at a severe risk. The frame may for example be a protecting cover, enclosing all or a part of the components included in the apparatus. For example, the frame may cover the first and second plates, and the flexible container in their entirety, and partially cover the delivery actuating unit, such that the medicament may exit the frame and reach the patient. The frame may comprise a hole or other type of opening to permit the medicament to exit the frame. The frame may be made of a non-magnetic material such as a hard plastic or similar.

According to another exemplifying embodiment, the apparatus is configured for one single use. This may allow for the customization of the apparatus according to the medication to be delivered. The size and strength of the magnets may then be adapted to the size of the dose to be delivered, and thus the size of the flexible container. Further, the size of the piercing element or sealed exit may be adapted for the same reasons. An advantage from this is that the apparatus is suitable for delivery of medicaments in a broad range of sizes, since the apparatus may be adapted for both smaller doses as well as bigger doses. A further advantage is that using the disclosed apparatus may remove a number of preparation steps that otherwise would have been required to be performed by a professional care taker when delivering the medicament. Instead, the preparatory steps may be performed before delivering the apparatus to the patient, such that the patient may be allowed to receive the medicament at home.

The medicament delivery devices described herein can be used for the treatment and/or prophylaxis of one or more of many different types of disorders. Exemplary disorders include, but are not limited to: rheumatoid arthritis, inflammatory bowel diseases (e.g. Crohn's disease and ulcerative colitis), hypercholesterolaemia, diabetes (e.g. type 2 diabetes), psoriasis, migraines, multiple sclerosis, anaemia, lupus, atopic dermatitis, asthma, nasal polyps, acute hypoglycaemia, obesity, anaphylaxis and allergies. Exemplary types of drugs that could be included in the medicament delivery devices described herein include, but are not limited to, small molecules, hormones, cytokines, blood products, antibodies, antibody-drug conjugates, bispecific antibodies, proteins, fusion proteins, peptibodies, polypeptides, pegylated proteins, protein fragments, protein analogues, protein variants, protein precursors, chimeric antigen receptor T cell therapies, cell or gene therapies, oncolytic viruses, or immunotherapies and/or protein derivatives. Exemplary drugs that could be included in the medicament delivery devices described herein include, but are not limited to (with non-limiting examples of relevant disorders in brackets): etanercept (rheumatoid arthritis, inflammatory bowel diseases (e.g. Crohn's disease and ulcerative colitis)), evolocumab (hypercholesterolaemia), exenatide (type 2 diabetes), secukinumab (psoriasis), erenumab (migraines), alirocumab (rheumatoid arthritis), methotrexate (amethopterin) (rheumatoid arthritis), tocilizumab (rheumatoid arthritis), interferon beta-1a (multiple sclerosis), sumatriptan (migraines), adalimumab (rheumatoid arthritis), darbepoetin alfa (anaemia), belimumab (lupus), peginterferon beta-1a' (multiple sclerosis), sarilumab (rheumatoid arthritis), semaglutide (type 2 diabetes, obesity), dupilumab (atopic dermatitis, asthma, nasal polyps, allergies), glucagon (acute hypoglycaemia), epinephrine (anaphylaxis), insulin (diabetes), atropine and vedolizumab (inflammatory bowel diseases (e.g. Crohn's disease and ulcerative colitis)) , ipilimumab, nivolumab, pembrolizumab, atezolizumab, durvalumab, avelumab, cemiplimab, rituximab, trastuzumab, adotrastuzumab emtansine, fam-trastuzumab deruxtecan-nxki, pertuzumab, transtuzumabpertuzumab, alemtuzumab, belantamab mafodotin-blmf, bevacizumab, blinatumomab, brentuximab vedotin, cetuximab, daratumumab, elotuzumab, gemtuzumab ozogamicin, 90-Yttrium-ibritumomab tiuxetan, isatuximab, mogamulizumab, moxetumomab pasudotox, obinutuzumab, ofatumumab, olaratumab, panitumumab, polatuzumab vedotin, ramucirumab, sacituzumab govitecan, tafasitamab, or margetuximab. Pharmaceutical formulations including, but not limited to, any drug described herein are also contemplated for use in the medicament delivery devices described herein, for example pharmaceutical formulations comprising a drug as listed herein (or a pharmaceutically acceptable salt of the drug) and a pharmaceutically acceptable carrier. Pharmaceutical formulations comprising a drug as listed herein (or a pharmaceutically acceptable salt of the drug) may include one or more other active ingredients, or may be the only active ingredient present.

Exemplary drugs that could be included in the medicament delivery devices described herein include, but are not limited to, an immuno-oncology or bio-oncology medications such as immune checkpoints, cytokines, chemokines, clusters of differentiation, interleukins, integrins, growth factors, enzymes, signaling proteins, pro-apoptotic proteins, anti-apoptotic proteins, T-cell receptors, B-cell receptors, or costimulatory proteins.

Exemplary drugs that could be included in the medicament delivery devices described herein include, but are not limited to, those exhibiting a proposed mechanism of action, such as HER-2 receptor modulators, interleukin modulators, interferon modulators, CD38 modulators, CD22 modulators, CCR4 modulators, VEGF modulators, EGFR modulators, CD79b modulators, Trop-2 modulators, CD52 modulators, BCMA modulators, PDGFRA modulators, SLAMF7 modulators, PD-1/PD-L1 inhibitors/modulators, B-lymphocyte antigen CD19 inhibitors, B-lymphocyte antigen CD20 modulators, CD3 modulators, CTLA-4 inhibitors, TIM-3 modulators, VISTA modulators, INDO inhibitors, LAG3 (CD223) antagonists, CD276 antigen modulators, CD47 antagonists, CD30 modulators, CD73 modulators, CD66 modulators, CDw137 agonists, CD158 modulators, CD27 modulators, CD58 modulators, CD80 modulators, CD33 modulators, APRIL receptor modulators, HLA antigen modulators, EGFR modulators, B-lymphocyte cell adhesion molecule modulators, CDw123 modulators, Erbb2 tyrosine kinase receptor modulators, mesothelin modulators, HAVCR2 antagonists, NY-ESO-1 OX40 receptor agonist modulators, adenosine A2 receptors, ICOS modulators, CD40 modulators, TIL therapies, or TCR therapies.

Exemplary drugs that could be included in the medicament delivery devices described herein include, but are not limited to, a multi-medication treatment regimen such as AC, Dose-Dense AC, TCH, GT, EC, TAC, TC, TCHP, CMF, FOLFOX, mFOLFOX6, mFOLFOX7, FOLFCIS, CapeOx, FLOT, DCF, FOLFIRI, FOLFIRINOX, FOLFOXIRI, IROX, CHOP, R-CHOP, RCHOP-21, Mini-CHOP, Maxi-CHOP, VR-CAP, Dose-Dense CHOP, EPOCH, Dose-Adjusted EPOCH, R-EPOCH, CODOX-M, IVAC, HyperCVAD, R-HyperCVAD, SC-EPOCH-RR, DHAP, ESHAP, GDP, ICE, MINE, CEPP, CDOP, GemOx, CEOP, CEPP, CHOEP, CHP, GCVP, DHAX, CALGB 8811, HIDAC, MOpAD, 7 + 3, 5 +2, 7 + 4, MEC, CVP, RBAC500, DHA-Cis, DHA-Ca, DHA-Ox, RCVP, RCEPP, RCEOP, CMV, DDMVAC, GemFLP, ITP, VIDE, VDC, VAI, VDC-IE, MAP, PCV, FCR, FR, PCR, HDMP, OFAR, EMA/CO, EMA/EP, EP/EMA, TP/TE, BEP, TIP, VIP, TPEx, ABVD, BEACOPP, AVD, Mini-BEAM, IGEV, C-MOPP, GCD, GEMOX, CAV, DT-PACE, VTD-PACE, DCEP, ATG, VAC, VelP, OFF, GTX, CAV, AD, MAID, AIM, VAC-IE, ADOC, or PE.

### Brief description of the drawings

One or more embodiments will be described, by way of example only, and with reference to the following figures, in which:
Figure 1 schematically illustrates an exploded view of an apparatus according to an embodiment of the present disclosure;
Figures 2A-C schematically illustrates a view of an apparatus in a first position and a delivery position;
Figures 3A-B illustrates a top view of a first and a second plate according to an exemplifying embodiment of the present disclosure.
Figure 4 illustrates a schematic view of a flexible container and a tube, and a sensor and an electromechanical valve arranged thereon;

### Detailed Description

The present disclosure is described in the following by way of a number of illustrative examples. It will be appreciated that these examples are provided for illustration and explanation only and are not intended to be limiting on the scope of the disclosure.

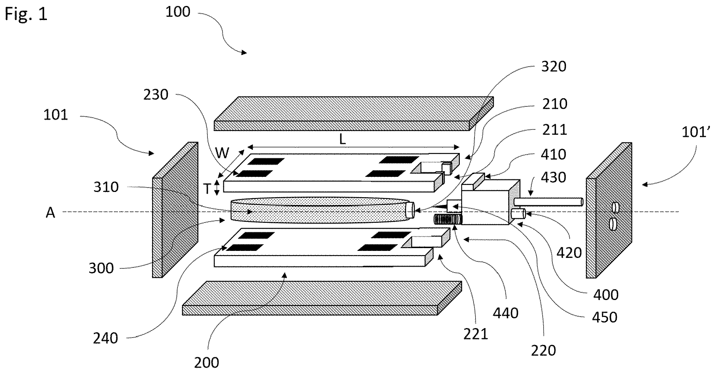

Figure 1 schematically illustrates an apparatus 100 according to an exemplifying embodiment of the present disclosure. The apparatus may comprise a frame 101 enclosing the apparatus 100, the frame having a top side, a bottom side, a back side and a front side 101'. The front side 101' may comprise one or more openings, configured to permit one or more internal components of the apparatus 100 to extend through the frame 101 to the environment outside the frame 101. For example, as illustrated in Fig. 1, the front side 101' comprises two openings, permitting a tube 430 and a releasing means 420 to extend through the front side 101' to the outer environment.

Further, the apparatus comprises a delivery unit 200. The delivery unit 200 comprises a first plate 210 and a second plate 220. The plates 210, 220 may have a substantially rectangular shape with a length L, a width W and a thickness T. The first 210 and second 220 plates may have the same dimensions L, W, T. The first and second plates 210, 220 are arranged on opposite sides along a longitudinal axis A. The first plate 210 may comprise a recess 211 arranged in one end of the plate. The recess may extend along a portion of the width W, and along a portion of the length L of the first plate 210. Further, the recess 211 may comprise a pair of protrusions 212. The protrusions 212 may protrude from the side of the recess 211 extending along the length L of the first plate 210. The protrusions 212 may also protrude from a side of the recess 211 extending along the width W of the first plate 210.

Similarly, the second plate 220 may comprise a recess 221 arranged in one end of the plate. The recess 221 may extend along a portion of the width W, and along a portion of the length L of the second plate 220. Further, the recess 221 may comprise a pair of grooves 222. The grooves 222 may protrude from the side of the recess 221 extending along the length L of the second plate 220. The grooves 222 may also protrude from a side of the recess 221 extending along the width W of the second plate 220.

It should be noted that the first and/or second plate can be formed in any suitable shape, e.g., oval, circular or triangle (when observe in the direction transverse to the longitudinal axis A. Dependent on the shape of the flexible container.

Further, the first 210 and second 220 plates comprise at least one first 230 and second 240 magnet. In the example illustrated in Fig. 1, the first plate 210 comprise four magnets 230. Similarly, the second plate 220 comprises four magnets 240. The four magnets 230 and the four magnets 240 may be distributed over a surface of the first 210 and second plates 220, respectively. The distribution of the four magnets 230 on the first plate 210, may correspond to a distribution of the four magnets 240 on the second plate 220. Further, the magnets 230, 240 may extend through the thickness T of the first and second plates 210, 220. The magnets 230, 240 may also be arranged on a surface of the first and second plates 210, 220. Referring to Fig. 1, a top surface of the first plate 210 is the surface facing the top side of the frame 101, and a bottom surface the surface facing the second plate 220. Similarly, a top surface of the second plate 220 is the surface facing the first plate 210, and a bottom surface the surface facing the bottom side of the frame 101. As seen in Fig. 1, the four magnets 230 and the four magnets 240 extends through the thickness T of the first 210 and second 220 plates.

The apparatus 100 further comprises a flexible container 300. The flexible container 300 comprises a medicament 310 to be delivered. The flexible container 300 may have the same length L as the first 210 and/or second 220 plates. The flexible container 300 is arranged along the longitudinal axis A. As seen in Fig. 1, the length of the flexible container 300 may have a shorter length than the first 210 and second plates 220, along axis A. Further, the flexible container 300 is arranged to be sandwiched between the first 210 and second 220 plates. The flexible container 300 may further comprise a sealed exit 320. The sealed exit 320 may be configured to permit the medicament 310 to exit the flexible container 300. As seen in Fig. 1, the sealed exit 320 is arranged in an end of the flexible container 300 corresponding to the end of the recesses 211 and 221 of the first 210 and second 220 plates.

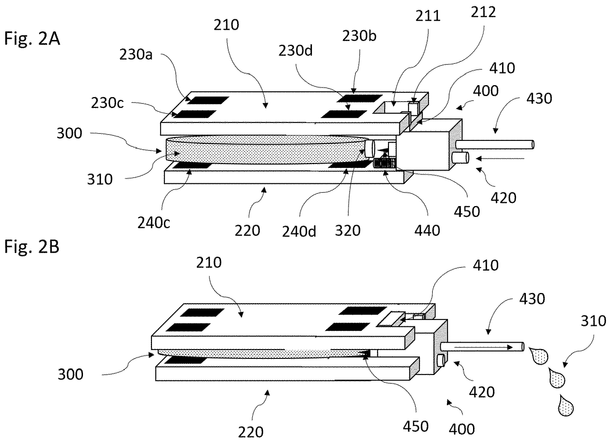

Adjacent the sealed exit 320 along the axis A, there is arranged a delivery actuating device 400, as seen in Fig. 1. The delivery actuating device 400 may be arranged to be partly engaged with the first 210 and second 220 plates, in a first position. The delivery actuating device 400 comprises a spacer 410 protruding from a side of the delivery actuating device 400. The spacer 410 is configured to engage with the first 210 and/or second 220 plate, to maintain said plates in a separated state. According to an example illustrated in Fig. 2A, the spacer 410 may engage with the protrusions 212 of the recess 211 in the first plate 210 when the plates 210, 220 are separated. The delivery actuating device 400 further comprises a releasing means 420. The releasing means 420 may be a push button as seen in Fig. 1. A tube 430 may protrude from the delivery actuating device 400. The tube 430 may extend through the delivery actuating device 400 and be arranged in connection with a piercing element 450. The delivery actuating device 400 may therefore provide a medicament path (not shown in the Figure) between the flexible container 300 through the delivery actuating device 400 to the receiving patient (not shown in the Figure). The tube 430 may be made of a transparent and/or flexible material. As illustrated in Fig. 1, the piercing element 450 may be arranged adjacent the sealed exit 320, along axis A. The piercing element 450 may be a needle. It will be appreciated that other possible embodiments, such as another type of sharp object, of the piercing element 450 exists. The piercing element 450 is further arranged to pierce the sealed exit 320 upon actuation of the releasing means 420. Thus, the piercing element 450 is advantageously arranged adjacent to the sealed exit 320.

As seen in Fig. 1, the delivery actuating means 400 may further comprise an elastic member 440. The elastic member 440 may be a compression spring. The elastic member 440 may be arranged along axis A, on a side of the delivery actuating device 400 arranged to be partly engaged with the first 210 and second 220 plates, as seen in Fig. 1. The elastic member 440 may thus be arranged on a side of the delivery actuating device 400 opposite to the releasing means 420. Upon actuation of the releasing means 420, the elastic member 440 may be compressed.

Figure 2A shows an apparatus 100 according to an exemplifying embodiment of the present disclosure, in a first position. The first 210 and second 220 plates are separated by the delivery actuating device 400 and the spacer 410. In one example, the pair of protrusions 212 rests on the spacer 410 in the first position. The delivery actuating device 400 may be partly inserted in the recess 221 of the second plate 220. According to an exemplifying embodiment illustrated in Fig. 2A, a width of the delivery actuating device 400 may be smaller than a width of the recesses 211 and 221 of the first 210 and second 220 plates.

Further, the delivery actuating device 400 may comprise a locking mechanism (not shown in the Figure) locking the delivery actuating device 400 and the first 210 and second 220 plates in the first position. Further, the flexible container 300 is sandwiched between the first 210 and second 220 plates in the first position. As seen in Fig. 2A, there is no pressure exerted on the flexible container 300 in the first position. The piercing element 450 is distanced from the sealed exit 320 such that the medicament 310 is remained in the flexible container 300. Further, the elastic member 440 is in a non-compressed state. The releasing means 420 is not actuated in the first position.

The four magnets 230a, 230b, 230c and 230d may be distributed on the first plate 210, towards each corner of the substantially rectangular shape. Similarly, the four magnets 240a (not shown in the Figure), 240b (not shown in the Figure), 240c and 240d may be distributed on corresponding positions on the second plate 220. Further, the magnets 230a and 240a may have opposite poles, same for 230b and 240b, 230c and 240c, and 230d and 240d, such that each "pair" of magnets attract each other.

Figure 2B shows the apparatus 100 from Fig. 2A, moved to a delivery position. The delivery actuating device 400 is actuated by a trigger of the releasing means 420. The triggering event may for example be a user or patient pushing a button, in the exemplifying embodiment wherein the releasing means 420 is a button, preferably, a push button. Upon triggering of the releasing means 420, the delivery actuating device 400 may slide towards the first 210 and second 220 plates. The delivery actuating device 400 may be slidably arranged along a length direction of the first 210 and second 220 plates. In the exemplifying embodiment wherein the delivery actuating device 400 is partly inserted in the recesses 211 and 221, the delivery actuating device 400 may be slidably arranged in said recesses 211 and 221. Upon triggering of the releasing means 420, the delivery actuating device 400 may therefore slide in the recesses 211 and 221. The spacer 410 may then slide out of its position engaged with the pair of protrusions 212. As seen in Fig. 2B, thereby the attracting force between the magnets 230a-d and 240a-d may therefore move the first 210 and second 220 plates towards each other. In a preferred example, the spacer comprises a convex surface or a beveled surface directed away from the flexible container. In this example, when the spacer 410 is moved toward the flexible container to move the delivery actuating device to the delivery position, the protrusion 212 will move along the convex surface or beveled surface to disengage from the first plate. Similar convex and beveled surface can be arranged at the end of the spacer that is configured to engage with the second plate.

A pressure P (not shown in the Figure) will be exerted on the flexible container 300 from the first 210 and second 220 plates as a result of the magnets 230a-d and 240a-d attracting each other. The pressure P exerted on the flexible container 300 will cause flexible container 300 to be compressed in height direction, the "height direction" herein defined as the direction in which the flexible container 300 extends between a bottom surface of the first plate 210, and a top surface of the second plate 220. Further, the flexible container may be expanded along a length direction and width direction of the first 210 and second 220 plates, as a result of it being compressed in a height direction. The strength of the magnets 230a-d and 240a-d together with the characteristics of the material of the flexible container 300 determines the expansion rate. An increased magnetic strength together with an increased flexibility in the material will increase an expansion rate, and thus also a delivery rate of the medicament 310. A decreased magnetic force together with a less flexible material will decrease the expansion rate, and thus a delivery rate of the medicament 310.

In one exemplifying embodiment, the piercing element 450 pierces the sealed exit 320 as a result of the flexible container 300 expanding due to the pressure P, thus removing the distance between the piercing element 450 and the sealed exit 320.

In another exemplifying embodiment, as seen in Fig. 2B, the piercing element 450 slides together with the delivery actuating device 400, thus removing the distance between the sealed exit 320 and the piercing element 450 by means of sliding. The piercing element 450 pierces the sealed exit 320 such that the medicament 310 is permitted to flow out from the flexible container 300. Further, upon piercing of the sealed exit 320, the piercing element 450 and the sealed exit 320 defines a medicament path along which the medicament 310 may exit the apparatus 100. The medicament path may be sealed, such that the delivery of the medicament 310 may be performed in a sterile manner.

According to an exemplifying embodiment, the sealed exit 320 may be made of a flexible material, and/or a material that has sealing characteristics, such that the piercing element 450 may pierce the sealed exit 320 such that it remains sealed after it has been pierced. The material may for example be a type of rubber.

As seen in Fig. 2B, the medicament 310 escapes the apparatus 100 through the tube 430. The size of the piercing element 450 may further impact the delivery rate of the medicament 310. An increased size of the piercing element 450 may yield an increased size of the opening in the sealed exit 320, thus increasing delivery rate of the medicament 310. Similarly, a decreased size of the piercing element 450 may yield a smaller opening in the sealed exit 320, thus decreasing the delivery rate.

Further, the releasing means 420, which in this exemplifying embodiment is a push button, remains in a pushed state in the delivery position. The user may then be ensured that the apparatus 100 has been actuated. The elastic member 440 (not shown in the Figure) is compressed upon triggering the releasing means 420. The elastic member 440 may be arranged on the delivery actuating device 400 such that the elastic member 440 abuts a bottom of the recess 221 in the length direction of the second plate 220. Therefore, the elastic member 440 may be compressed upon movement from the first position to the delivery position. Since the elastic member 440 is arranged between the delivery actuating device 400 and the delivery unit 200, the elastic member 440 may have a compression load to overcome in order to compress the elastic element 440.

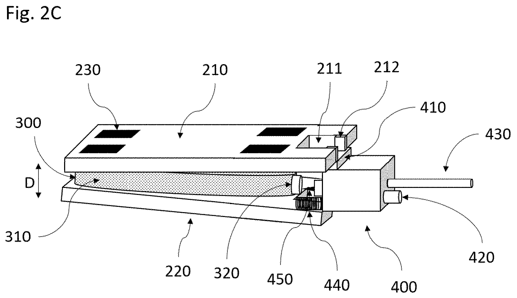

Figure 2C shows the apparatus 100 according to an exemplifying embodiment of the present disclosure. Figure 2C illustrates an alternative installation configuration of the delivery unit 200. The first plate 210 and the second plate 220 are arranged with a varying distance D to each other. The distance between the first 210 and second 220 plates may vary along a length direction of said plates 210, 220. As seen in Fig. 2C, the distance between the plates 210, 220 in the end where the delivery actuating device 400 is arranged, is greater than the distance in the opposite end of the plates 210, 220. This embodiment may be advantageous in that one delivery actuating device 400 sufficient to maintain the plates 210, 220 in a separated state. Further, the magnets 230a and 230c and 240a and 240c are arranged with a smaller distance than the magnets 230b and 230d along with 240b and 240d. Thus, the attracting force of these magnets is greater than the other magnets, resulting in a motion of the first 210 and second 220 plates that will press the medicament 310 in the flexible container 300 towards the sealed exit 320. This may ensure that no medicament 310 gets stuck inside the flexible container 300.

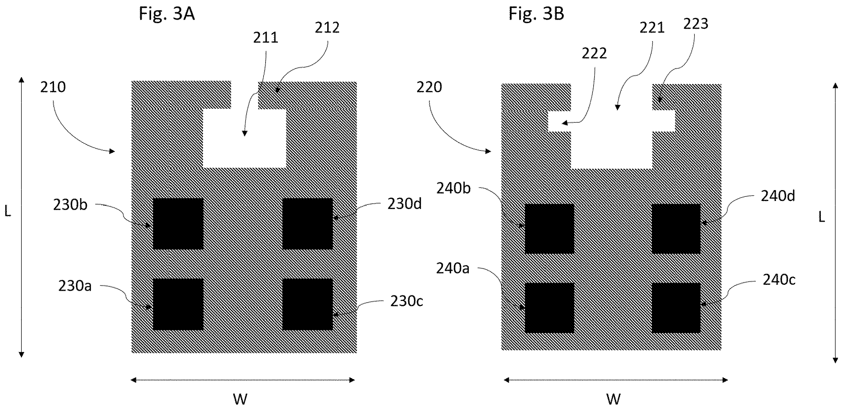

Figures 3A-B illustrates a top view of the first 210 and second 220 plates according to an exemplifying embodiment of the present disclosure. In the present embodiment, the first 210 and second 220 plates have a substantially rectangular shape, with a length L and a width W. The length L and width W is the same for the first plate 210 and the second plate 220. Further, the recess 211 of the first plate 210 is shown in Figure 3A. The recess 211 may have a substantially rectangular shape. It will be appreciated that other shapes of the recess 211 are possible. Further, the protrusions 212 of the recess 211 may have a substantially rectangular shape and/or a shape corresponding to a shape of the spacer 410.

The recess 221 of the second plate 220 is shown in Figure 3B. The recess 221 may have a substantially rectangular shape. It will be appreciated that other shapes of the recess 221 are possible. Further, the grooves 222 of the recess 221 may have a substantially rectangular shape. The grooves 222 is configured to retain the delivery actuating unit 400 in the delivery position. The grooves 222 is configured to receive the protrusion of the spacer 410 when the delivery actuating device is in the delivery position. In this example, the spacer 410 is contact with a body part 223 of the second plate when the delivery actuating device is in the first position, and is moved into the groove 222 when the delivery actuating device is in the delivery position.

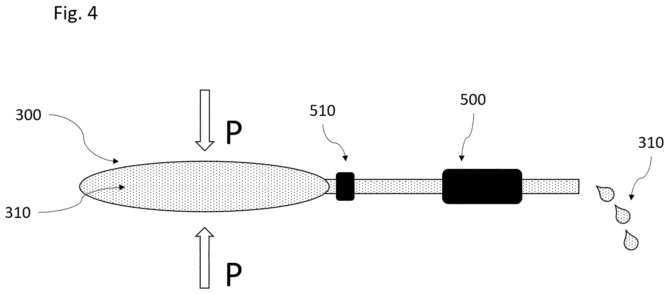

Figure 4 illustrates a schematic view of a flexible container 300 and a tube 430, comprising a sensor 500 and an electromechanical valve 510 arranged thereon. When a pressure P is exerted on the flexible container 300 from the first 210 and second 220 plates (not shown in the Figure), the medicament 310 is pressed out from the flexible container 310 through the tube 430. To further control a delivery rate of the medicament 310, a sensor 500 may be arranged on the tube 430. The sensor 430 measures a flow rate of the medicament 310. Further, an electromechanical valve 510 may be arranged on the tube 430. The sensor 500 and the electromechanical valve 510 may be connected. A measured flow rate from the sensor 500 may be communicated to the electromechanical valve 510 in order to control a flow rate (i.e. delivery rate) of the medicament 310. The sensor 500 may be arranged close to an opening of the tube 430 where the medicament 310 exits the medicament path. The sensor 500 may also be arranged close to an opposite end of the tube 430, where the medicament 430 enters the medicament path. Furthermore, the sensor 500 and the electromechanical valve 510 may be connected to a control unit (not shown in the Figure) for processing of the sensor measurement and the communication/signal sent to the electromechanical valve 510.

While the present disclosure is susceptible to various modifications and alternative forms, specific embodiments are shown and described by way of example in relation to the drawings, with a view to clearly explaining the various advantageous aspects of the present disclosure. It should be understood, however, that the detailed description herein and the drawings attached hereto are not intended to limit the disclosure to the particular form disclosed.

## Claims

1. An apparatus for delivery of a medicament, the apparatus comprising:
a flexible container (300) containing the medicament;
a delivery unit comprising;
a first plate (210) extending along a longitudinal axis (A); wherein the first plate (210) comprises at least one first magnet;
a second plate (220) extending along a longitudinal axis (A); wherein the first plate; wherein the second plate (220) comprises at least one second magnet; wherein
a portion of the flexible container (300) is positioned between the first plate (210) and the second plate (220) in a direction transverse to the longitudinal axis (A);
a delivery actuating device (400) movably arranged between a first position where the delivery actuating device (400) is at least partially engaged with the first and second plate (210, 220), such that the flexible container (300) is prevented from being pressed between the first plate (210) and the second plate (220) and a delivery position where the delivery actuating device (400) is disengaged from the first and second plate (210, 220) in the first position, such that the flexible container (300) can be pressed between the first plate (210) and the second plate (220);
**characterized in that** the delivery actuating device (400) comprises a piercing element (450) extending in the direction of the longitudinal axis (A), wherein the piercing element (450) is spaced apart from the flexible container (300) when the delivery actuating device (400) is in the first position; and wherein the piercing element (450) is positioned into the flexible container (300) when the delivery actuating device (400) is in the delivery position.

2. Apparatus according to claim 1, wherein the at least one first magnet and at least one second magnet have opposite magnetic poles.

3. Apparatus according to any of claims 1 to 2, wherein the flexible container (300) further comprises a sealed exit where the medicament can be expelled from the exit.

4. Apparatus according to claim 3, wherein the sealed exit comprises a pierceable seal.

5. Apparatus according to any one of the preceding claims, wherein the delivery actuating device (400) further comprises a spacer (410) at least partially arranged between the first and second plates (210, 220) in the direction transverse to the longitudinal axis (A) when the delivery actuating device (400) is in the first position; and wherein the spacer (410) is spaced apart from the first plate (210) and the second plate (220) when the delivery actuating device (400) is in the delivery position.

6. Apparatus according to claim 5, wherein the spacer (410) is made of a non-magnetic material.

7. Apparatus according to claim 5 or 6, wherein the delivery actuating device (400) comprises a button connected to the spacer (410); and wherein the delivery actuating device (400) is configured to be moved from the first position to the delivery position when the button (420) is manually moved.

8. Apparatus according to claim 7, wherein the button (420) is a push button (420).

9. Apparatus according to any one of claims 5 to 8, further comprising an elastic element (440) extending in the direction of the longitudinal axis (A), between the delivery unit and the delivery actuating device (400); wherein the elastic element (440) is configured to support the spacer (410) in the first position.

10. Apparatus according to claim 9, wherein the elastic element (440) is a spring (440).

11. Apparatus according to claim 9 or 10, wherein the elastic element extends in the direction of the longitudinal axis (A) between a first end and a second end; wherein the first end is adjacent to either the first plate (210) or the second plate (220); and wherein the second end is adjacent to the spacer (410).

12. Apparatus according to any one of the preceding claims , wherein the piercing element (450) is a needle.

13. Apparatus according to claim 12, when dependent on claim 9 or claim 10, wherein the piercing element (450) is attached to the button (420).

## Patentansprüche

1. Vorrichtung zur Abgabe eines Medikaments, wobei die Vorrichtung umfasst:
einen flexiblen Behälter (300), der das Medikament enthält;
eine Abgabeeinheit, umfassend;
eine erste Platte (210), die sich entlang einer Längsachse (A) erstreckt; wobei die erste Platte (210) mindestens einen ersten Magneten umfasst;
eine zweite Platte (220), die sich entlang einer Längsachse (A) erstreckt; wobei die erste Platte; wobei die zweite Platte (220) mindestens einen zweiten Magneten umfasst; wobei
ein Abschnitt des flexiblen Behälters (300) in einer Richtung quer zu der Längsachse (A) zwischen der ersten Platte (210) und der zweiten Platte (220) angeordnet ist;
eine Abgabebetätigungsvorrichtung (400), die beweglich zwischen einer ersten Position, in der die Abgabebetätigungsvorrichtung (400) zumindest teilweise mit der ersten und der zweiten Platte (210, 220) in Eingriff steht, sodass verhindert wird, dass der flexible Behälter (300) zwischen der ersten Platte (210) und der zweiten Platte (220) gepresst wird, und einer Abgabeposition, in der die Abgabebetätigungsvorrichtung (400) von der ersten und der zweiten Platte (210, 220) in der ersten Position außer Eingriff steht, sodass der flexible Behälter (300) zwischen der ersten Platte (210) und der zweiten Platte (220) gepresst werden kann, angeordnet ist;
**dadurch gekennzeichnet, dass** die Abgabebetätigungsvorrichtung (400) ein Durchstechelement (450) umfasst, das sich in Richtung der Längsachse (A) erstreckt, wobei das Durchstechelement (450) von dem flexiblen Behälter (300) beabstandet ist, wenn sich die Abgabebetätigungsvorrichtung (400) in der ersten Position befindet; und wobei das Durchstechelement (450) in den flexiblen Behälter (300) positioniert wird, wenn sich die Abgabebetätigungsvorrichtung (400) in der Abgabeposition befindet.

2. Vorrichtung nach Anspruch 1, wobei der mindestens eine erste Magnet und mindestens ein zweiter Magnet entgegengesetzte Magnetpole aufweisen.

3. Vorrichtung nach einem der Ansprüche 1 bis 2, wobei der flexible Behälter (300) ferner einen abgedichteten Ausgang umfasst, wo das Medikament aus dem Ausgang ausgestoßen werden kann.

4. Vorrichtung nach Anspruch 3, wobei der abgedichtete Ausgang eine durchstechbare Dichtung umfasst.

5. Vorrichtung nach einem der vorstehenden Ansprüche, wobei die Abgabebetätigungsvorrichtung (400) ferner einen Abstandshalter (410) umfasst, der zumindest teilweise zwischen der ersten und der zweiten Platte (210, 220) in der Richtung quer zu der Längsachse (A) angeordnet ist, wenn sich die Abgabebetätigungsvorrichtung (400) in der ersten Position befindet; und wobei der Abstandshalter (410) von der ersten Platte (210) und der zweiten Platte (220) beabstandet ist, wenn sich die Abgabebetätigungsvorrichtung (400) in der Abgabeposition befindet.

6. Vorrichtung nach Anspruch 5, wobei der Abstandshalter (410) aus einem nichtmagnetischen Material hergestellt ist.

7. Vorrichtung nach Anspruch 5 oder 6, wobei die Abgabebetätigungsvorrichtung (400) einen Knopf umfasst, der mit dem Abstandshalter (410) verbunden ist; und wobei die Abgabebetätigungsvorrichtung (400) konfiguriert ist, um von der ersten Position in die Abgabeposition bewegt zu werden, wenn die Taste (420) manuell bewegt wird.

8. Vorrichtung nach Anspruch 7, wobei die Taste (420) eine Drucktaste (420) ist.

9. Vorrichtung nach einem der Ansprüche 5 bis 8, ferner umfassend ein elastisches Element (440), das sich in der Richtung der Längsachse (A) zwischen der Abgabeeinheit und der Abgabebetätigungsvorrichtung (400) erstreckt; wobei das elastische Element (440) konfiguriert ist, um den Abstandshalter (410) in der ersten Position zu stützen.

10. Vorrichtung nach Anspruch 9, wobei das elastische Element (440) eine Feder (440) ist.

11. Vorrichtung nach Anspruch 9 oder 10, wobei sich das elastische Element in Richtung der Längsachse (A) zwischen einem ersten Ende und einem zweiten Ende erstreckt; wobei das erste Ende entweder an die erste Platte (210) oder an die zweite Platte (220) angrenzt; und wobei das zweite Ende an den Abstandshalter (410) angrenzt.

12. Vorrichtung nach einem der vorstehenden Ansprüche, wobei das Durchstechelement (450) eine Nadel ist.

13. Vorrichtung nach Anspruch 12, wenn abhängig von Anspruch 9 oder Anspruch 10, wobei das Durchstechelement (450) an der Taste (420) befestigt ist.

## Revendications

1. Appareil destiné à la délivrance d'un médicament, l'appareil comprenant :
un récipient souple (300) contenant le médicament ;
une unité de délivrance comprenant ;
une première plaque (210) s'étendant le long d'un axe longitudinal (A) ; dans lequel la première plaque (210) comprend au moins un premier aimant ;
une seconde plaque (220) s'étendant le long d'un axe longitudinal (A) ; dans lequel la première plaque ; dans lequel la seconde plaque (220) comprend au moins un second aimant ; dans lequel
une partie du récipient souple (300) est positionnée entre la première plaque (210) et la seconde plaque (220) dans une direction transversale à l'axe longitudinal (A) ;
un dispositif d'actionnement de délivrance (400) agencé de manière mobile entre une première position où le dispositif d'actionnement de délivrance (400) est au moins partiellement en prise avec la première et la seconde plaque (210, 220), de telle sorte que le récipient souple (300) est empêché d'être pressé entre la première plaque (210) et la seconde plaque (220) et une position de délivrance où le dispositif d'actionnement de délivrance (400) est désolidarisé de la première et de la seconde plaque (210, 220) dans la première position, de telle sorte que le récipient souple (300) peut être pressé entre la première plaque (210) et la seconde plaque (220) ;
**caractérisé en ce que** le dispositif d'actionnement de délivrance (400) comprend un élément de perçage (450) s'étendant dans la direction de l'axe longitudinal (A), dans lequel l'élément de perçage (450) est espacé du récipient souple (300) lorsque le dispositif d'actionnement de délivrance (400) est dans la première position ; et dans lequel l'élément de perçage (450) est positionné dans le récipient souple (300) lorsque le dispositif d'actionnement de délivrance (400) est dans la position de délivrance.

2. Appareil selon la revendication 1, dans lequel l'au moins un premier aimant et au moins un second aimant ont des pôles magnétiques opposés.

3. Appareil selon l'une quelconque des revendications 1 à 2, dans lequel le récipient souple (300) comprend en outre une sortie scellée où le médicament peut être expulsé de la sortie.

4. Appareil selon la revendication 3, dans lequel la sortie scellée comprend un scellage perçable.

5. Appareil selon l'une quelconque des revendications précédentes, dans lequel le dispositif d'actionnement de délivrance (400) comprend en outre un élément d'espacement (410) au moins partiellement agencé entre les première et seconde plaques (210, 220) dans la direction transversale à l'axe longitudinal (A) lorsque le dispositif d'actionnement de délivrance (400) est dans la première position ; et dans lequel l'élément d'espacement (410) est espacé de la première plaque (210) et de la seconde plaque (220) lorsque le dispositif d'actionnement de délivrance (400) est dans la position de délivrance.

6. Appareil selon la revendication 5, dans lequel l'élément d'espacement (410) est fabriqué dans un matériau non magnétique.

7. Appareil selon la revendication 5 ou 6, dans lequel le dispositif d'actionnement de délivrance (400) comprend un bouton relié à l'élément d'espacement (410) ; et dans lequel le dispositif d'actionnement de délivrance (400) est conçu pour être déplacé de la première position à la position de délivrance lorsque le bouton (420) est manuellement déplacé.

8. Appareil selon la revendication 7, dans lequel le bouton (420) est un bouton poussoir (420).

9. Appareil selon l'une quelconque des revendications 5 à 8, comprenant en outre un élément élastique (440) s'étendant dans la direction de l'axe longitudinal (A), entre l'unité de délivrance et le dispositif d'actionnement de délivrance (400) ; dans lequel l'élément élastique (440) est conçu pour supporter l'élément d'espacement (410) dans la première position.

10. Appareil selon la revendication 9, dans lequel l'élément élastique (440) est un ressort (440).

11. Appareil selon la revendication 9 ou 10, dans lequel l'élément élastique s'étend dans la direction de l'axe longitudinal (A) entre une première extrémité et une seconde extrémité ; dans lequel la première extrémité est adjacente soit à la première plaque (210) soit à la seconde plaque (220) ; et dans lequel la seconde extrémité est adjacente à l'élément d'espacement (410).

12. Appareil selon l'une quelconque des revendications précédentes, dans lequel l'élément de perçage (450) est une aiguille.

13. Appareil selon la revendication 12, prise en dépendance de la revendication 9 ou de la revendication 10, dans lequel l'élément de perçage (450) est attaché au bouton (420).
